# EUROPÄISCHE PATENTSCHRIFT

(11) **EP 2 839 290 B1**
(45) Veröffentlichungstag und Bekanntmachung des Hinweises auf die Patenterteilung: **06.03.2019**
(21) Anmeldenummer: 13722278.2
(22) Anmeldetag: 17.04.2013
(51) Int. Cl.: G01N 33/68, C12Q 1/52

(54) **VERFAHREN ZUR PRÄSYMPTOMATISCHEN DIAGNOSTIK VON ZÖLIAKIE UND GLUTENSENSITIVITÄT**
METHOD FOR PRESYMPTOMATIC DIAGNOSIS OF COELIAC DISEASE AND GLUTEN SENSITIVITY
PROCÉDÉ DE DIAGNOSTIC PRÉ-SYMPTOMATIQUE DE LA MALADIE C LIAQUE ET DE LA SENSIBILITÉ AU GLUTEN

(30) Priorität: 17.04.2012 DE 102012007510; 17.04.2012 DE 102012007508
(43) Veröffentlichungstag der Anmeldung: 25.02.2015
(73) Patentinhaber: Aeneas GmbH & Co. KG, 55234 Wendelsheim (DE)
(72) Erfinder: MATTHIAS, Torsten, 55234 Wendelsheim (DE); PFEIFFER, Sascha, 55545 Bad Kreuznach (DE); PRAGER, Kai, 55545 Bad Kreuznach (DE); MEESTERS, Christian, 55262 Heidesheim am Rhein (DE); JEREMIAS, Patricia, 55597 Woellstein (DE)
(74) Vertreter: Fritzsche, Thomas
(86) Internationale Anmeldenummer: PCT/EP2013/001140
(87) Internationale Veröffentlichungsnummer: WO 2013/156156

(56) Entgegenhaltungen:
- WO-A2-2008/053310
- WO-A2-2008/053310
- SKOVBJERG H ET AL: "Deamidation and cross-linking of gliadin peptides by transglutaminases and the relation to celiac disease", BIOCHIMICA ET BIOPHYSICA ACTA. MOLECULAR BASIS OF DISEASE, AMSTERDAM, NL, Bd. 1690, Nr. 3, 5. November 2004 (2004-11-05), Seiten 220-230, XP004614704, ISSN: 0925-4439, DOI: 10.1016/J.BBADIS.2004.06.009
- SKOVBJERG H ET AL: "Gliadin is a good substrate of several transglutaminases: Possible implication in the pathogenesis of coeliac disease", SCANDINAVIAN JOURNAL OF GASTROENTEROLOGY, Bd. 37, Nr. 7, Juli 2002 (2002-07), Seiten 812-817, XP009171985, ISSN: 0036-5521
- DEKKING ET AL: "Microbial transglutaminases generate T cell stimulatory epitopes involved in celiac disease", JOURNAL OF CEREAL SCIENCE, ACADEMIC PRESS LTD, XX, Bd. 47, Nr. 2, 27. Februar 2008 (2008-02-27), Seiten 339-346, XP022500544, ISSN: 0733-5210
- BIZZARO N ET AL: "Cutting-Edge Issues in Celiac Disease and in Gluten Intolerance", CLINICAL REVIEWS IN ALLERGY & IMMUNOLOGY, HUMANA PRESS INC, NEW YORK, Bd. 42, Nr. 3, 23. Dezember 2010 (2010-12-23), Seiten 279-287, XP035055779, ISSN: 1559-0267, DOI: 10.1007/S12016-010-8223-1
- SKOVBJERG H ET AL: "Deamidation and cross-linking of gliadin peptides by transglutaminases and the relation to celiac disease", BIOCHIMICA ET BIOPHYSICA ACTA. MOLECULAR BASIS OF DISEASE, AMSTERDAM, NL, vol. 1690, no. 3, 5 November 2004 (2004-11-05), pages 220-230, XP004614704, ISSN: 0925-4439, DOI: 10.1016/J.BBADIS.2004.06.009
- SKOVBJERG H ET AL: "Gliadin is a good substrate of several transglutaminases: Possible implication in the pathogenesis of coeliac disease", SCANDINAVIAN JOURNAL OF GASTROENTEROLOGY, INFORMA HEALTHCARE, UK, vol. 37, no. 7, 1 July 2002 (2002-07-01), pages 812-817, XP009171985, ISSN: 0036-5521
- DEKKING ET AL: "Microbial transglutaminases generate T cell stimulatory epitopes involved in celiac disease", JOURNAL OF CEREAL SCIENCE, ACADEMIC PRESS LTD, GB, vol. 47, no. 2, 27 February 2008 (2008-02-27), pages 339-346, XP022500544, ISSN: 0733-5210
- BIZZARO N ET AL: "Cutting-Edge Issues in Celiac Disease and in Gluten Intolerance", CLINICAL REVIEWS IN ALLERGY & IMMUNOLOGY, HUMANA PRESS INC, NEW YORK, vol. 42, no. 3, 23 December 2010 (2010-12-23), pages 279-287, XP035055779, ISSN: 1559-0267, DOI: 10.1007/S12016-010-8223-1

## Beschreibung

Die Zöliakie, auch glutensensitive Enteropathie oder Sprue genannt, ist eine chronische Entzündung des Dünndarms mit oft ausgedehnter Zerstörung der Darmepithelzellen, was zu einem Malabsorptions-Syndrom und/oder immunologischen Begleiterkrankungen führen kann.

Die Entzündung wird hierbei, bei entsprechender genetischer Disposition, aufgrund einer Überempfindlichkeit gegen Gluten, ein Sammelbegriff für die in vielen Getreidearten vorhandenen sog. "Klebeeiweiße" aktiviert. Im Weizen wird das immunogene Prolamin (die alkohollösliche Fraktion) Gliadin genannt. Homologe Prolamine sind auch in Roggen (Secaline), Gerste (Hordeine) und Hafer (Avenine) bekannt. Bei Menschen mit entsprechender Prädisposition können diese Peptidfragmente zu einer komplexen Reaktion der Darmschleimhaut und des Immunsystems führen.

Unter Gluten sind ebenso insbesondere Glutenderivate, Gliadin oder Gliadinhomologe Extrakte, nicht synthetisch erzeugte oder synthetisch Erzeugte Gliadinpeptide und chemische Derivate derselben unter "Gliadinpeptid" zu versehen.

Bestimmte Gliadinpeptide oder homologe Prolaminpeptidfragmente werden von antigenpräsentierenden Zellen aufgenommen, prozessiert und auf HLA-Moleküle geladen oder binden direkt an HLA-Moleküle, hauptsächlich an HLA-DQ2 oder HLA-DQ8. Verstärkt wird diese Bindung dadurch, dass die in der häufig im Peptid vorhandene Aminosäure Glutamin deamidiert wird. Diese Deamidierung wird hauptsächlich durch das Enzym Gewebs-Transglutaminase (tTg), insbesondere durch die Gewebs-Transglutaminase 2 (tTg2) katalysiert. Daneben katalysiert das Enzym auch eine Transamidierung, durch die ein Vernetzen der Umgebungspeptide und Proteine induziert wird. Durch die Deamidierung von Gliadinpeptiden wird eine hochaffine Bindung zwischen dem Gliadinpeptid und zum Beispiel HLA-DQ2 garantiert.

Dieser Peptid-HLA-Komplex wiederum bindet nun an CD4+-T-Helferzellen, was wiederum zur Aktivierung der inflammatorischen Zytokin-Kaskade führt. Dadurch wird vermehrt die Produktion von verschiedenen entzündungsauslösenden Botenstoffen hervorgerufen, wie beispielsweise Interferon-gamma, TNF-alpha, Interleukin-6 und Interleukin-2.

Im Verlauf der Entzündung werden verschiedene Antikörper gebildet, welche zum Beispiel gegen Gliadin selbst (Gliadin-Antikörper) oder auch gegen die Gewebs-Transglutaminase (tTg-Antiköper) gerichtet sind. Letztlich endet der Entzündungsprozess in der Apoptose der Enterozyten (Saumzellen des Dünndarmepithels), was zu einem mehr oder weniger ausgeprägten Verlust von Dünndarmzotten (Zottenatrophie) führt. Dadurch ist die Dünndarmschleimhaut nicht mehr in der Lage genügend Nährstoffe vom Darm in die Blutbahn zu transferieren, wodurch es im Endeffekt zu einem Malabsorptions-Syndrom kommt.

Die Prävalenz (Anzahl der zum Untersuchungszeitpunkt Kranken/Anzahl der "betrachteten" Individuen) der Zöliakie oder einheimischen Sprue beträgt 1:100, neuere Studien gehen aber von einer Prävalenz von ca. 1:50 aus. Alles in allem tritt die Zöliakie oder einheimische Sprue mit einer Häufigkeit von 1% bis 3% innerhalb der kaukasischen Bevölkerungsgruppe auf.

Die Zöliakie hat zwei Manifestationsgipfel, einen im Säuglingsalter mit ca. 6 bis 18 Monaten, ein bis drei Monate nachdem Cerealien in die Ernährung mit aufgenommen wurden und einen zweiten Gipfel im Alter von ca. 30 bis 40 Jahren.

Die häufigsten Symptome der Zöliakie oder Sprue sind durch die Verdauungsstörung bedingte chronische Diarrhö, Eisenmangel mit oder ohne Anämie und Osteopenie. Bei betroffenen Kindern ist oft eine Gedeihstörung zu beobachten. Seltenere Symptome sind zum Beispiel chronische Müdigkeit, Nervosität, Osteoporose, Osteomalazie, Angststörungen, Depressionen, Muskelkrämpfe und sekundäre Laktose-Intoleranz. Des Weiteren können auch verschiedene Begleiterkrankungen auftreten, wie zum Beispiel ein selektiver IgA-Mangel, Dermatitis herpetiformis, Diabetes Typ I, und gastrointestinale Malignome.

Die Diagnose der Zöliakie oder Sprue wird bevorzugt oder üblicherweise mittels einer serologischen Diagnostik und/oder mittels Histologie von Dünndarmbiopsien durchgeführt. Die serologische Diagnostik der Zöliakie oder Sprue wird üblicherweise auf das Vorhandensein von Antikörpern gegen ein Fremdantigen, wie beispielsweise Gliadin (anti-Gliadin-Antikörper) oder deamidiertes Gliadinpeptid (anti-DGP-Antikörper) und gegen Autoantigene des Endomysiums (anti-EMA-Antikörper), insbesondere jedoch gegen Gewebstransglutaminase 2 (anti-tTg2-Antikörper) oder einem Komplex aus Gewebstransglutaminase und Gliadin (anti-tTg/Gliadinpeptid-Komplex-Antikörper) getestet.

Autoantikörper gegen Endomysial-Antigene sind hochspezifisch und lassen sich bei über 90% der Patienten mit einer Zöliakie bzw. einheimischen Sprue nachweisen. Hierbei handelt es sich um einen indirekten Immunofluoreszenztest an Gewebsschnitten aus Affenösophagus. Die Anti-Endomysium-Konzentrationen spiegeln das histologische Erscheinungsbild wider: je höher die Antikörpertiter sind, desto ausgeprägter ist auch die Zottenatrophie. Jedoch ist der Anti-Endomysium-Nachweis mittels der Immunofluoreszenz-Technik nicht ohne erheblichen Aufwand durchführbar, da dieser Test besondere technische Fähigkeiten des Anwenders fordert, einen hohen Zeitbedarf benötigt und relativ rare biologische Material (Affenösophagusgewebe) beansprucht wird.

Der Nachweis von Antikörpern gegen Gliadin war historisch gesehen die erste Möglichkeit, um eine Zöliakie bzw. einheimische Sprue mithilfe eines Antikörper-Testverfahrens zu erkennen. Jedoch sind anti-Gliadin-Antikörper nicht spezifisch genug, um die einheimische Sprue in einem befriedigenden Maße zu detektieren. Die Detektionssensitivität konnte durch Auffinden von anti-deamidierten-Gliadinpeptid(DGP)-Antikörpern verbessert werden. Eine weitere Verbesserung zur Diagnose und Verlaufskontrolle dieser Erkrankung war das Auffinden von anti-Gewebs-Transglutaminase-Antikörpern.

Bei der serologischen Diagnostik der Zöliakie werden üblicherweise Anti-Endomysiale Antikörper nachgewiesen.

Ein zentrales Element der Zöliakie-Diagnostik war und ist zum Teil heute immer noch eine histologische Untersuchung einer Dünndarmbiopsie, wobei das histopathologische Spektrum dabei von einer minimalen Vermehrung intraepithelialer Lymphozyten bis zur vollständigen Zottenatrophie reichen kann. Dabei sind charakteristische histopathologische Elemente: Vermehrung intraepithelialer Lymphozyten sowie Vermehrung der Lymphozyten und Plasmazellen in der Lamina propria, häufig vermischt mit Eosinophilen; verminderte Zottenlänge, Vertiefung der Krypten; verminderte Zotten: Krypten-Relation (Normal >4-5:1); erhöhte Mitosenzahl; abnorme Enterozyten (kuboide anstatt zylindrische Zellen, Verlust der basalen Kern-Polarität, Verlust des Bürstensaums).

Das histologische Grading wird üblicherweise nach der von Marsh vorgeschlagenen Klassifikation vorgenommen.

**Tabelle : Einteilung der Marsh Kriterien [A.Vècsei et al. 2011, p. 6]**

| Typ | Krypten | Zotten |
|---|---|---|
| 0 | normal | normal |
| 1 | normal | normal |
| 2 | hyperplastisch | normal |
| 3a | hyperplastisch | leicht verkürzt |
| 3b | hyperplastisch | stark verkürzt |
| 3c | hyperplastisch | fehlen ganz |

Aufgrund der Diagnose von Symptomen, histologischen Befunden und serologischen Befunden, sowie genetischen und klinischen Informationen lässt sich die Zöliakie oder einheimische Sprue in verschiedene klinische Untergruppen unterteilen. Diese reichen von der sogenannten "silent Sprue" über die "latente Sprue" bis hin zur manifesten oder klassischen Sprue.

Eine frühzeitige Diagnose der Zöliakie oder einheimischen Sprue ist für den Krankheitsverlauf von entscheidender Bedeutung. Dadurch, dass mit einer gesicherten und rechtzeitigen Diagnose die Erkrankung durch konsequente Einhaltung einer glutenfreien Diät in Remission gehalten werden kann und die Risiken von Begleit- und/oder Folgekrankheiten, wie zum Beispiel das erhöhte Malignom-Risiko, verhindert werden kann.

Es ist deshalb von größtem Interesse, Nachweistests für die Zöliakie oder einheimische Sprue weiter zu verbessern. Für einen schnellen, leicht durchführbaren und preiswerten Test kommen vor allen Dingen serologische Diagnostik-Verfahren in Betracht.

Die EP 0 912 898 B1 lehrt zum Beispiel ein immunologisches Nachweisverfahren von Antikörpern, die gegen Gewebs-Transglutaminase (tTg) gerichtet sind. Bei diesem Diagnose-Verfahren werden Antikörper gegen Gewebs-Transglutaminase (tTG) aus Körperflüssigkeiten durch eine Immunreaktion mit Gewebe-Transglutaminase nachgewiesen, wobei die Immunreaktion nicht mit einem Gewebeschnitt eines tierischen oder menschlichen Gewebes durchgeführt wird.

Das Dokument SKOVBJERG H et al. "Deamidation and cross-linking of gliadin peptides by transglutaminases and the relation to celiac disease", Biochimica et Biophysica Acta - Molecular Basis of Disease, Amsterdam, NL, Bd. 1690, Nr. 3, 5. November 2004 (2004-11-05), Seiten 220-230, XP004614704, ISSN: 0925-4439, DOI: 10.1016/J.BBADIS.2004.06.009, offenbart, dass humane Gewebstransglutaminase sowie Streptoverticillium-Transglutaminase in der Lage sind, ein synthetisches Epitop eines Gliadinpeptides zu deamidieren.

Die Druckschrift SKOVBJERG H et al. "Gliadin is a good substrate of several transglutaminases: possible implication in the pathogenesis of coeliac disease.", Scandinavian journal of Gastroenterology, informa healthcare, UK, Bd. 37, Nr. 7, 1. Juli 2002 (2002-07-01), Seiten 812-817, XP009171985, ISSN: 0036-5521, offenbart eine Methode zur Aktivitätsmessung verschiedener Transglutaminasen. Als Substrat wird hierbei Solpro 300, ein proteolytisch aufgespaltenes Gluten, verwendet, anhand dessen die Transglutaminaseaktivitäten von Meerschweinchenlebertransglutaminase, Streptoverticillium-Transglutaminase und der Transglutaminase aus *Phytophthora cactorum* getestet wurde.

Die Druckschrift DEKKING et al.: "Microbial transglutaminases generate T cell stimulatory epitopes involved in celiac disease", JOURNAL OF CEREAL SCI-ENCE, ACADEMIC PRESS LTD, GB, Bd. 47, Nr. 2, 27. Februar 2008 (2008-02-27), Seiten 339 - 346, XP022500544, ISSN: 0733-5210, beschreibt Untersuchungen zur Deamidierung von Gluten durch die mikrobielle Transglutaminase Streptoverticillium mobaraense. Hierbei konnte gezeigt werden, dass die mikrobielle Transglutaminase ein breiteres Substratspektrum aufweist als die Gewebstransglutaminase und sowohl synthetische als auch natürliche Glutenpeptide deamidieren kann.

Die WO 2008/053310 A2 beschreibt die Verwendung eines enzymatischen Verfahrens zur Behandlung von Mehl, Grieß und Proteinextrakten aus Zerealien, welche dafür bekannt sind, eine pathogene Immunantwort in Patienten mit CD (Celiac Disease) hervorzurufen. Das Verfahren wendet hierbei die katalytische Aktivität von mikrobieller Transglutaminase an, um damit die Toxizität von Gluten oder ähnlichen Produkten aus dieses Zerealien zu reduzieren oder komplett zu eliminieren.

Die Druckschrift BIZZARO N et al. "Cutting-edge issues in celiac disease and in gluten intolerance", Clinical Reviews in Allergy & Immunology, Humana Press Inc., New York, Bd. 42, Nr. 3, 23. Dezember 2010 (2010-12-23), Seiten 279-287, XP035055779, ISSN: 1559-0267, DOI: 10.1007/S12016-010-8223-1, beschreibt relevante Aspekte in Bezug auf die Labordiagnose von Zöliakie und von Glutenintoleranz in Bezug auf die geeignetsten Testkombinationen für eine frühe und akkurate Diagnose der Krankheit. Unter anderem beschreibt die Druckschrift Tests für die Detektion von Antikörpern gegen Komplexe aus Gewebstransglutaminase und Gliadin.

Außerdem sind Testverfahren bekannt, welche Antikörper detektieren können, die gegen einen Komplex aus Gewebetransglutaminase (tTG) und Gliadinpeptiden gerichtet sind. Solche Testverfahren werden zum Beispiel von der Firma AESKU.Diagnostics angeboten. Es hat sich gezeigt, dass mit diesen Testverfahren eine Antikörperbildung, welche noch vor allen anderen Tests aus dem Stand der Technik auftritt, nachgewiesen werden kann.

Darüber hinaus wird auch eine Glutenunverträglichkeit bzw. -Sensitivität beschrieben, welche nicht durch Zöliakie hervorgerufen wird. Diese Erkrankung ist von der Zöliakie zu unterscheiden und ist weder eine Autoimmunerkrankung, noch eine Weizenallergie. Beides muss zu ihrer Diagnose zuerst ausgeschlossen werden. Darüber hinaus muss festgestellt werden, ob bei einer glutenfreien Diät eine Besserung stattfindet. Für diese Erkrankung sind bislang keine Biomarker gefunden worden.

Ziel der vorliegenden Erfindung ist es, einen diagnostischen Test bereitzustellen, welcher in der Lage ist, bereits vor Entwicklung von Symptomen, d. h. präsymptomatisch, Zöliakie oder Sprue sowie eine Glutensensitivität zu detektieren und somit eine frühe Diagnose bzw. Therapiekontrolle der Zöliakie bzw. Sprue oder allgemeinen Glutenunverträglichkeiten bzw. Glutensensitivität zu ermöglichen.

Dieses Ziel wird durch die in den Ansprüchen definierten Merkmale erreicht. Erfindungsgemäß wurde gefunden, dass zur Diagnose und/oder Therapiekontrolle der Zöliakie oder Sprue bzw. der Glutensensitivität anstatt der Gewebstransglutaminase (tTG) mindestens ein funktioneller, immunologisch reaktiver Komplex aus mikrobieller Transglutaminase (mTG) oder Teile derselben und Gliadin oder Teilpeptide desselben verwendet wird. Im Folgenden wird unter dem Begriff Gliadin nicht nur Gliadin selbst, sondern auch Gliadinpeptide sowie Teilpeptide davon verstanden.
Überraschenderweise wurde nämlich gefunden, dass bereits in einem frühen Stadium der zuvor genannten Erkrankungen Antikörper aufzufinden sind, welche an einen Komplex binden, der aus mikrobieller Transglutaminase und Gliadin gebildet ist, wobei der immunologisch reaktive Komplex zum Nachweis von Antikörpern aus einer Probe dient und diese Antikörper nachweisbar sind, noch bevor Antikörper nachweisbar sind, die gegen einen Komplex aus Gewebstransglutaminase und Gliadin gebildet werden. Dies ist umso überraschender, da die mikrobielle Transglutaminase eine völlig andere Sequenz aufweist als die humane Gewebstransglutaminase (tTg).
Analog zur Funktion der Gewebstransglutaminase wird auch bei der Generierung des erfindungsgemäß verwendeten Komplexes davon ausgegangen, dass das Verhältnis von De- zu Transamidierung 4:1 beträgt und der erfindungsgemäß verwendete Komplex nicht zwangsläufig eine mit der Transglutaminase kovalent komplexierte Molekülspezies aufweist. Der Komplex wird vorzugsweise durch eine Inkubation von Gliadinpeptiden und mikrobieller Transglutaminase gebildet. Die Einzelkomponente mTg zeigt keine immunologische Aktivität in humanen Proben, Gliadinpeptide sind nach Stand der Technik bekannte Antigene und zeigen somit immunologische Aktivität in humanen Patienten-Proben. Die immunologische Aktivität ist jedoch nach Komplexbildung zwischen Gliadinpeptiden und mTg deutlich höher als die der Einzelkomponenten zusammen.

Analog zum Komplex aus tTg2 und Gliadinpeptiden wird auch beim erfindungsgemäß verwendeten Komplex davon ausgegangen, dass die Verteilung der vernetzten Gliadinpeptide und dem Enzym stochastisch erfolgt, welches durch die Lichtstreu-Messungen belegt ist. Diese Komplexe beinhalten die mikrobielle Transglutaminase und zeigen immunologische Aktivität auf, die geeignet ist in Kompetitionstests die Epitope aus tTg2 mit Gliadinpeptiden abzufangen.

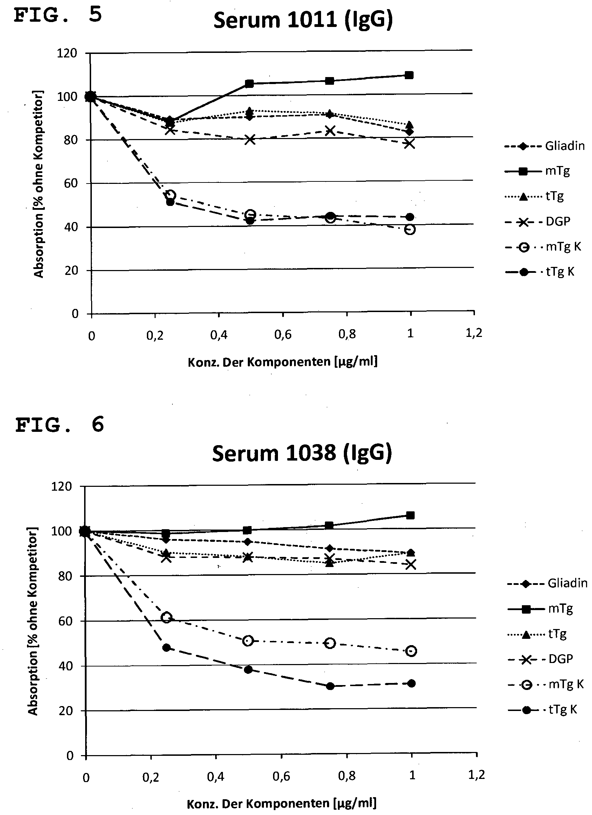

Belegt wird dies durch Kompetitionstests, welche mit verschiedenen Patientenseren durchgeführt wurden (vgl. Fig.5).

Die vorliegende Erfindung bezieht sich auch auf Antikörper, die an den erfindungsgemäß verwendeten Komplex aus mikrobieller Transglutaminase und Gliadinpeptiden oder an immunologisch aktive Teile dieses Komplexes binden. Antikörper im Sinne der Erfindung umfassen sowohl polyklonale und/oder monoklonale Antikörper und/oder Aptamere. Unter einem Antikörper wird dabei ein Protein verstanden welches eine oder mehrere spezifische Antigenbindungsstellen aufweist. Der Fachmann ist ohne unzumutbaren Aufwand in der Lage, einen Antikörper zu generieren, welcher spezifisch an den erfindungsgemäß verwendeten Komplex aus mikrobieller Transglutaminase und Gliadinpeptiden oder an immunologisch aktive Teile dieses Komplexes bindet. Die entsprechenden Techniken und Herangehensweisen sind dem Fachmann aus täglicher Laborpraxis bekannt. Die erfindungsgemäßen Antikörper können z.B. auch dadurch generiert werden, dass die im Serum von Zöliakie- oder Sprue-Patienten bzw. von glutensensitiven Patienten vorhandenen Antikörper mittels eines erfindungsgemäß verwendeten Komplexes isoliert, gereinigt und damit zugänglich gemacht werden. Ohne daran gebunden zu sein, wird vermutet, dass die Antikörper an ein Neoepitop in der Nähe des aktiven Zentrums des Enzyms mTgase binden. Dazu kann z. B. der erfindungsgemäß verwendete Komplex an einen Träger gekoppelt vorliegen, der beladene Träger wird dann mit Zöliakie- oder Sprue-Patientenserum in Kontakt gebracht. Unspezifisch gebundene Bestandteile des Serums werden entfernt und die spezifisch an den erfindungsgemäß verwendeten Komplex gebundenen Antikörper werden daraufhin eluiert.

Die vorliegende Erfindung bezieht sich auch auf eine Verwendung des Komplexes aus mikrobieller Transglutaminase und Gliadin oder deren Teilpeptide zum in vitro Nachweis von Antikörpern, welche an diesen Komplex binden. Die erfindungsgemäße Verwendung zeichnet sich dadurch aus, dass:
1. ein erfindungsgemäß verwendeter Komplex mit einer Probe in vitro in Kontakt gebracht wird; und
2. dass an den erfindungsgemäß verwendeten Komplex gebundene Antikörper nachgewiesen werden.
Für die Zwecke der vorliegenden Erfindung wird unter dem Begriff "in vitro" jede Umgebung verstanden, die nicht innerhalb eines ganzen Organismus, beispielsweise eines menschlichen oder tierischen Körpers liegt.
Unter einer Probe wird eine zu untersuchende Zusammensetzung verstanden. Bevorzugt handelt es sich bei der Probe um biologisches oder medizinisches Material, also Material, das von einem Organismus, von Bestandteilen eines Organismus oder von Zellen gewonnen wird. Das Material kann, bevor es als Probe im erfindungsgemäßen Verfahren eingesetzt wird, weiteren Behandlungsschritten unterzogen werden, z. B. um das Material in einen Zustand zu versetzten, in dem es sich als Probe für das Verfahren besonders eignet. Besonders bevorzugt handelt es sich bei der Probe um Material, das aus einer Körperflüssigkeit gewonnen wurde oder aus einer Körperflüssigkeit besteht. Bevorzugte Körperflüssigkeiten sind Blut, Plasma, Serum, Lymphe, Synovialflüssigkeit, Urin, Stuhl, Interstitialflüssigkeit, Speichel, Schweiß, Spinalflüssigkeit, Muttermilch und/oder Tränenflüssigkeit. Besonders bevorzugt sind solche Körperflüssigkeiten, in denen Antikörper in hoher Konzentration zu finden sind.
Bei der erfindungsgemäßen Verwendung wird ein erfindungsgemäß verwendeter Komplex mit einer zu untersuchenden Probe in vitro in Kontakt gebracht. Der Schritt des Inkontaktbringens dient dazu, dass ggf. in der Probe enthaltene Antikörper die Möglichkeit haben, an ein Epitop des erfindungsgemäß verwendeten Komplexes zu binden. Dazu wird dieser Schritt unter Bedingungen und in einer Umgebung durchgeführt, die eine spezifische Antigen-Antikörper-Bindung erlauben. Dem Fachmann sind geeignete Bedingungen bekannt. Bevorzugt umfassen diese Bedingungen eine flüssige Umgebung (optional auch feste Trägermaterialien) und/oder das Inkontaktbringen bei einer Temperatur von > 0°C bis < 60°C. Das Inkontaktbringen wird bevorzugt über einen Zeitraum durchgeführt, der eine Ausbildung einer spezifischen Antigen-Antikörper-Bindung zwischen dem erfindungsgemäß verwendeten Komplex und ggf. in der Probe enthaltenem für den Komplex aus mikrobieller Transglutaminase und Gliadin oder deren immunologisch aktiven Teilpeptiden spezifischen Antikörper erlaubt.
In einem nachfolgenden Schritt der erfindungsgemäßen Verwendung wird ein Antikörper nachgewiesen, der an den Komplex aus mikrobieller Transglutaminase und Gliadinpeptiden spezifisch gebunden ist. Der Nachweis des spezifisch an den erfindungsgemäß verwendeten Komplex gebundenen Antikörpers kann beispielsweise dadurch erfolgen, dass nach dem Inkontaktbringen, solche Bestandteile der Probe entfernt werden, z. B. durch einen oder mehrere Wasch-, Reinigungs- oder Isolierungsschritte, die nicht an den Komplex aus mikrobieller Transglutaminase und Gliadin oder deren Teilpeptide dieses Komplexes gebunden sind. Danach kann ein spezifischer Nachweis von Antikörpern mittels üblicher, dem Fachmann bekannter Methoden durchgeführt werden. Dabei kann der Nachweis in einem oder in mehreren Schritten erfolgen. Bei diesen Mitteln zum spezifischen Nachweis von Antikörpern kann es sich beispielsweise selbst um Antikörper handeln. Der Nachweis kann dann z. B. durch eine Farbreaktion erfolgen, die direkt oder indirekt durch die Mittel zum Nachweis von Antikörpern vermittelt oder ausgelöst wird. Beispielsweise können Antikörper zum Nachweis von spezifischen Antikörpern an funktionale Gruppen oder Moleküle gebunden sein (z. B. Enzyme), die in der Lage sind unter bestimmten Bedingungen eine Farbreaktion auszulösen oder zu vermitteln.
Bei einer erfindungsgemäßen Verwendung kann der Komplex aus mikrobieller Transglutaminase und Gliadin oder deren Teilpeptide während einem, mehrerer oder aller Schritte der Verwendung an einen Träger immobilisiert vorliegen. Unter Immobilisierung wird hierbei jede Kopplung, Bindung oder anderweitige Assoziierung zwischen Komplex und Träger verstanden, die dazu führt, dass Komplex und Träger nicht getrennt voneinander bewegt werden können. Als Träger können beispielsweise Moleküle und/oder Oberflächen eingesetzt werden, die derart ausgestaltet sind, dass sie mit dem Komplex reversibel oder irreversibel verbindbar sind. Dazu können Träger und/oder erfindungsgemäß verwendete Gliadine und/oder erfindungsgemäße verwendete Komplexe funktionale Gruppen aufweisen, die eine Verbindung zwischen Gliadinen und/oder erfindungsgemäß verwendetem Komplex und Träger unterstützen und/oder ermöglichen. Beispielhaft seien als Träger Moleküle erwähnt, wie BSA oder Oberflächen, wie sie von Mikropartikeln, Nanopartikeln oder magnetischen Beads angeboten werden oder Oberflächen von ausgewählten Membranen, Polymeren (z. B. Polystyren) oder solche Oberflächen umfassende Mikrotiterplatten oder Teststreifen. Dem Fachmann sind geeignete Träger und Möglichkeiten zur Verbindung von Proteinkomplexen und Trägern bekannt.

Insbesondere kann das erfindungsgemäße Verfahren als Immunassay-Verfahren durchgeführt werden, geeignete Immunassay-Verfahren sind beschrieben in "Labor und Diagnose", S. 756 ff. (ISBN 3980521567***)***
Bevorzugt kann das erfindungsgemäße Verfahren als ELISA-Verfahren (ELISA = enzyme-linked immunosorbent assay,) durchgeführt werden, geeignete ELISA-Techniken sind beispielsweise beschrieben in "Labor und Diagnose", S. 1470 ff. (ISBN 3980521567***).*** Dazu kann eine Probe mit einem an einen Träger immobilisierten erfindungsgemäß verwendeten Komplex in Kontakt gebracht werden, ggf. werden ungebundene Bestandteile teilweise oder im Wesentlichen entfernt, anschließend wird zum Nachweis von an dem erfindungsgemäß verwendeten Komplex gebundenem Probenantikörper ein an eine funktionale Gruppe gekoppelter oder koppelbarer Antikörper verwendet. Der Nachweis erfolgt in der Regel über eine optisch nachweisbare Reaktion.

Der Antikörper zum Nachweis kann beispielsweise spezifisch sein für Antikörper eines bestimmten Organismus oder eines bestimmten Ursprungs und/oder für eine bestimmte Form des Antikörpers, bevorzugt für einen bestimmten Isotyp z. B. Antikörper vom Typ IgA, IgM, IgE und/oder IgG, ganz besonders bevorzugt für humane IgA, IgM, IgE und/oder humane IgG.
Die erfindungsgemäße Verwendung kann aber auch in anderen Formaten durchgeführt werden, so z. B. als RIA (radio-immuno-assay), als Immunoassay (z. B. sog. Lineblots oder ELISAs) oder in flüssigkeitsbasierten Verfahren wie HTRF (homogenous time resolved fluorescence). Die erfindungsgemäße Verwendung kann auch in sogenannten Multiplexassays (Assays mit anderen Biomarkern) in den genannten technischen Verfahren kombiniert eingesetzt werden.
Die vorliegende Verwendung eignet sich zum Nachweis von Antikörpern gegen einen erfindungsgemäß verwendeten Komplex, insbesondere zum Nachweis von Antikörpern vom Typ IgA, IgG, IgM und/oder IgE bevorzugt zum Nachweis von Antikörpern humanen Ursprungs.
Die erfindungsgemäße Verwendung kann zur Diagnose, insbesondere zur serologischen Diagnose, bevorzugt zur Diagnose von Zöliakie und/oder einheimischer Sprue und/oder tropischer Sprue und/oder frühzeitigen Detektion von allgemeinen Gluten Unverträglichkeiten und/oder Dermatitis herpetiformis verwendet werden.
Die vorliegende Erfindung umfasst auch einen Kit zur Bestimmung bzw. Diagnose und/oder Therapiekontrolle von Zöliakie oder Sprue sowie von Glutensensitivität, wobei der Kit eine mikrobielle Transglutaminase oder Teile derselben, welche in einem Komplex mit Gliadin oder Teilpeptiden desselben vorliegt, sowie zusätzliche Mittel zum spezifischen Nachweis von an den immunologisch reaktiven Komplex gebundenen Antikörpern aus einer Probe umfasst. Zusätzlich kann der Kit eine Anweisung zur Verwendung des Kits und/oder zur Durchführung der mit dem Kit durchführbaren erfindungsgemäßen Verwendung enthalten.

Bevorzugt ist der Kit als ELISA oder insbesondere als Streifentest ausgeführt. Das heißt, der erfindungsgemäß verwendete Kit umfasst den erfindungsgemäß verwendeten Komplex und ggf. weitere Bestandteile zur Durchführung der erfindungsgemäßen Verwendung in einer Form, die sich zur Durchführung im ELISA- und/oder Streifentestformat eignet. Insbesondere kann der Kit den erfindungsgemäß verwendeten Komplex gekoppelt an einen Teststreifen umfassen.
Der erfindungsgemäß verwendete Kit kann ggf. weitere Bestandteile zur Durchführung der erfindungsgemäßen Verwendung enthalten. Solche Bestandteile können beispielsweise Reaktionsgefäße, Filter, Lösungen, proteinmodifizierende Enzyme und/oder andere Mittel umfassen. Insbesondere kann der erfindungsgemäße Kit Mittel zum Nachweis von Antikörpern enthalten, bevorzugt von humanen Antikörpern des IgA-, IgM-, IgE- und/oder des IgG-Typs.
Insbesondere eignet sich der erfindungsgemäß verwendete Kit zur Verwendung in der Diagnose, bevorzugt in der serologischen Diagnose, ganz besonders bevorzugt in der Diagnose von Zöliakie, Sprue, Dermatitis herpetiformis und/oder Glutensensitivität.
Der erfindungsgemäß verwendete Kit kann ggf. weitere Mittel zur Durchführung der erfindungsgemäßen Verwendung und/oder zur Einteilung der Zöliakie oder Sprue in spezifische Untergruppen (s.o., z. B. Silent, latent oder etablierte Zöliakie) enthalten. Solche Mittel können beispielsweise tTg-Antikörper, tTg/Gliadinpeptid-Komplex-Antikörper, DGP-Antikörper, Gliadin-Antikörper usw. umfassen.
**FIG. 1** zeigt ein paarweises Alignment der Aminosäuresequenzen von mikrobieller Transglutamase (mTG) aus Streptomyces mobaraensis und von humaner Gewebs-Transglutamase 2 (TG2) unter Verwendung des "strecher"-Algorithmus. Der Sequenzvergleich wurde mittels des im Internet frei zugänglichen "pairwise alignment tool" EMBOSS (von EMBL-EBI) durchgeführt.
**FIG. 2** zeigt ein paarweises Alignment der Aminosäuresequenzen von mikrobieller Transglutamase (mTG) aus Streptomyces mobaraensis und von humaner Gewebstransglutamase 2 (TG2) unter Verwendung des "needle"-Algorithmus. Der Sequenzvergleich wurde mittels des im Internet frei zugänglichen "pairwise alignment tool" EMBOSS (von EMBL-EBI) durchgeführt.
**FIG. 3** zeigt in Form eines Diagramms die Ergebnisse eines Kompetitionstests von Serum 1011 (IgA), wobei die Absorption in Abhängigkeit der Konzentration der Kompetitoren dargestellt ist. Die einzelnen Kompetitoren sind der Legende des Diagrammes zu entnehmen.
**FIG. 4** zeigt in Form eines Diagramms die Ergebnisse eines Kompetitionstests von Serum 1038 (IgA), wobei die Absorption in Abhängigkeit der Konzentration der Kompetitoren dargestellt ist. Die einzelnen Kompetitoren sind der Legende des Diagrammes zu entnehmen.
**FIG. 5** zeigt in Form eines Diagramms die Ergebnisse eines Kompetitionstests von Serum 1011 (IgG), wobei die Absorption in Abhängigkeit der Konzentration der Kompetitoren dargestellt ist. Die einzelnen Kompetitoren sind der Legende des Diagrammes zu entnehmen.
**FIG. 6** zeigt in Form eines Diagramms die Ergebnisse eines Kompetitionstests von Serum 1038 (IgG), wobei die Absorption in Abhängigkeit der Konzentration der Kompetitoren dargestellt ist. Die einzelnen Kompetitoren sind der Legende des Diagrammes zu entnehmen.

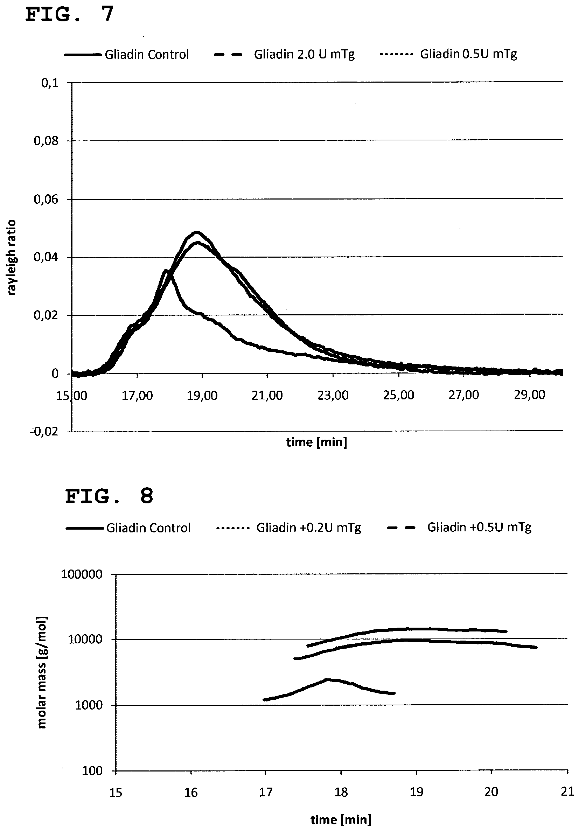

**FIG. 7** zeigt in Form eines Diagramms die Ergebnisse einer Messung über AF4-MALS zur Bildung von Komplexen aus Gliadin und mikrobieller Transglutaminase, wobei die sog. "rayleight"- Ratio in Abhängigkeit der Zeit dargestellt ist. Die einzelnen Komponenten der Messung sind der Legende des Diagrammes zu entnehmen.
**FIG. 8** zeigt in Form eines Diagramms die Ergebnisse einer Messung über AF4-MALS zur Bildung von Komplexen aus Gliadin und mikrobieller Transglutaminase, wobei die die molare Masse in g/mol in Abhängigkeit der Zeit dargestellt ist. Die einzelnen Komponenten der Messung sind der Legende des Diagrammes zu entnehmen.

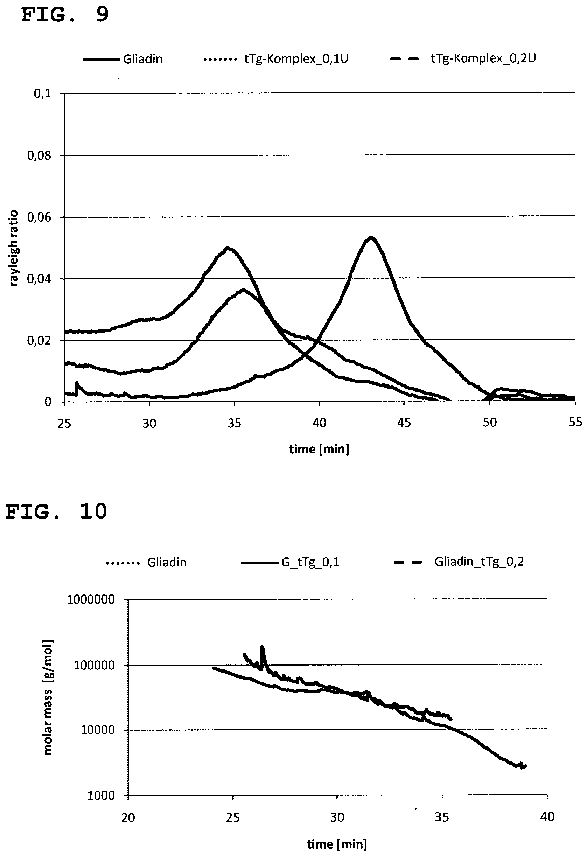

**FIG. 9** zeigt in Form eines Diagramms die Ergebnisse einer Messung über SEC-MALS zur Bildung von Komplexen aus Gliadin und Gewebe-Transglutaminase, wobei die sog. "rayleight"- Ratio in Abhängigkeit der Zeit dargestellt ist. Die einzelnen Komponenten der Messung sind der Legende des Diagrammes zu entnehmen.
**FIG. 10** zeigt in Form eines Diagramms die Ergebnisse einer Messung über SEC-MALS zur Bildung von Komplexen aus Gliadin und Gewebe-Transglutaminase, wobei die molare Masse in g/mol in Abhängigkeit der Zeit dargestellt ist. Die einzelnen Komponenten der Messung sind der Legende des Diagrammes zu entnehmen.

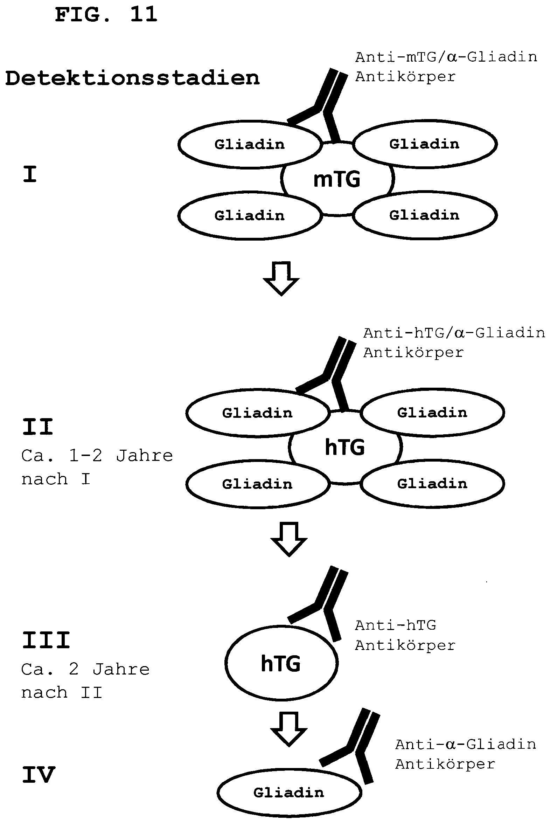

**FIG. 11** zeigt eine schematische Einteilung verschiedener Detektionsstadien der Zöliakie oder Spure. Grundlage ist hierbei das zeitlich verschiedene (erst)auftreten verschiedener Diagnose-Antikörper.

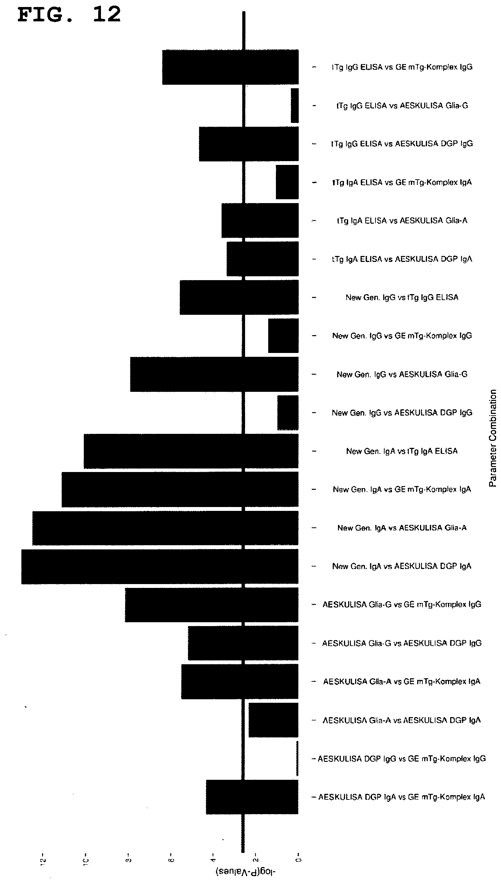

**FIG. 12** zeigt: -log(P-Werte) des Wilcoxon-Mann-Whitney-Tests zur Überprüfung der vermuteten Abfolge des Antikörperauftretens.

### Beispiele

### Aminosäurenseauenzalianments

Es soll gezeigt werden, dass die die mikrobiellen Transglutaminase (mTG) von *Streptomyces mobaraensis* und der humanen Gewebstransglutaminase (TG2) nur geringe Ähnlichkeit auf der Sequenzebene haben.

Zu diesem Zweck wurden die in der Datenbank, Uniprot (http://www.uniprot.org), vorhandenen Sequenzen *Q5UCB5* (mTG) und *P21980* (TG2) verglichen. Es wurde ein Sequenzvergleich mittels des im Internet vorhandenen "pairwise alignment tool" EMBOSS (von EMBL-EBI; http://www.ebi.ac.uk/Tools/psa/) durchgeführt. Es wurden die Algorithmen "needle" bzw. "strecher" verwendet.

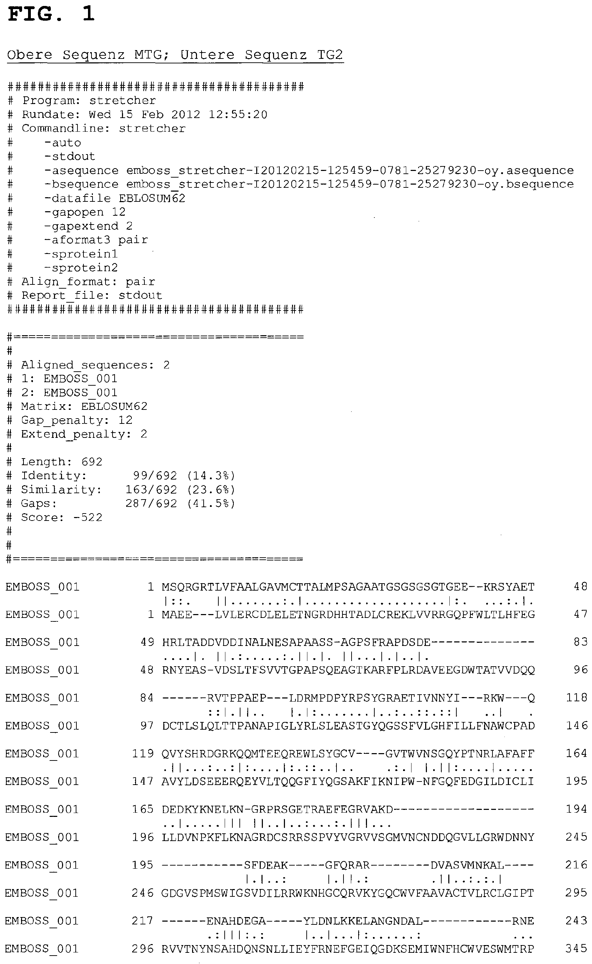

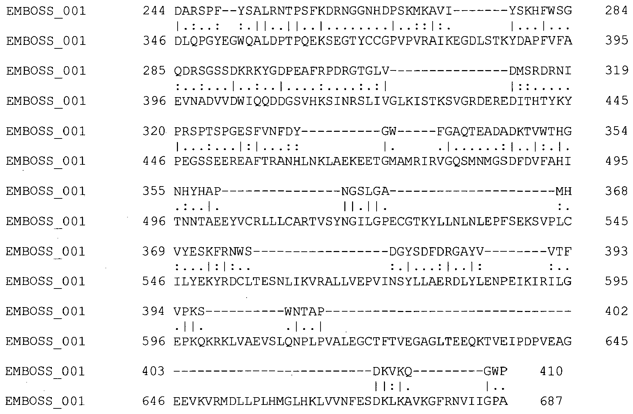

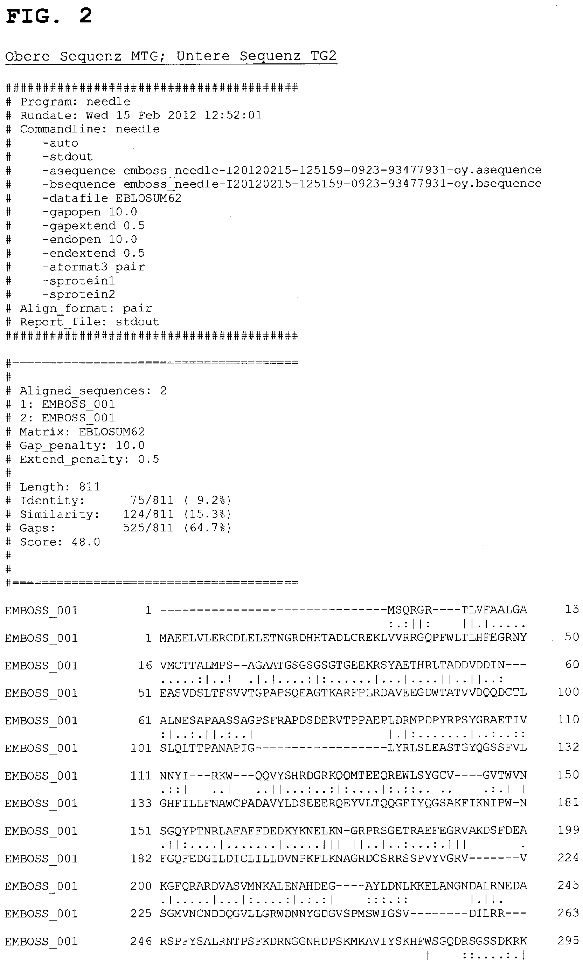

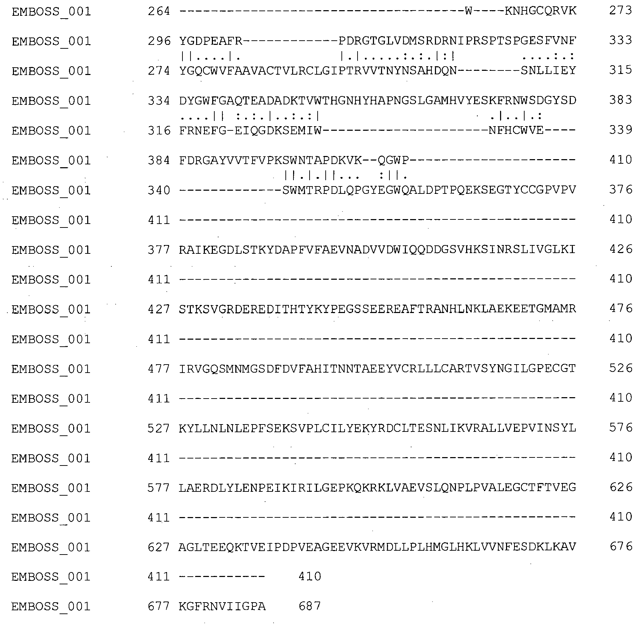

Wie aus FIG. 1 bzw. FIG. 2 ersichtlich ist, besteht nahezu keine Homologie zwischen den verwendeten Sequenzen, unabhängig davon welcher Algorithmus verwendet wird. Das paarweise Alignment zwischen mikrobieller Transglutaminase von *Streptomyces mobaraensis* und humaner Gewebe-Transglutaminase besitzt bei Verwendung des "stretcher"-Algorithmus nur eine Ähnlichkeit von 23,6% und eine Identität von 14,3%, und bei Verwendung des "needle"-Algorithmus lediglich eine Ähnlichkeit von 15,3% und eine Identität von 9,2%.

### Kompetitionstests

In Kompetitionsversuchen wird getestet, ob ein Zusammenhang zwischen dem gebildeten Komplex der humanen Gewebe-Transglutaminase mit Gliadin und dem erfindungsgemäß verwendeten Komplex besteht. Hierzu werden Patientenseren, die Antikörper gegen den Komplex aus Gewebe-Transglutaminase und Gliadin enthalten mit den Einzelkomponenten, sowie dem gebildeten erfindungsgemäßen Komplex aus mikrobieller Transglutaminase und Gliadin vermischt und auf eine mit dem Komplex aus Gewebe-Transglutaminase und Gliadin beschichteten Mikrotiterplatte gegeben. Über die Intensität einer Farbreaktion lässt sich nun ermitteln, wie stark jede Komponente zum Testergebnis beiträgt, also welcher reaktive Anteil der Antikörper an die jeweiligen Komponenten bindet. Wenn die Antikörper nicht nur an den Komplex aus humaner Gewebe-Transglutaminase und Gliadin, sondern auch an die anderen Kompetitoren binden, werden diese Antikörper weggefangen (und können somit nicht mehr an die Mikrotiterplatte binden) und die Farbreaktion fällt im Vergleich zum Referenzwert niedriger aus. Um einen Referenzwert zu erhalten, wird das Patientenserum direkt auf die mit dem Komplex aus humaner Gewebe-Transglutaminase und Gliadin beschichtete Platte gegeben.

Zur Durchführung des Kompetitionstests wurde ein AESKUKLISA tTg New Generation Kit der Firma AESKU.Diagnostics verwendet, welcher für eine separate quantitative und qualitative Bestimmung von IgA und/oder IgG Antikörpern, gerichtet gegen einen Komplex aus Gewebe-Transglutaminase und Gliadin, in humanen Seren geeignet ist.

### Konzentrationen der Einzelkomponenten:

Die Komponenten mTG, DGP, tTG sowie Gliadin wurden in den Konzentrationen 0; 0,125; 0,25; 0,5; 0,75 sowie 1,0 µg/ml eingesetzt (Doppelbestimmung der jeweiligen Konzentration wurde durchgeführt).

Das Serum wurde jeweils mit dem jeweiligen Kompetitor inkubiert und anschließend im Standard ELISA nach Angaben des Herstellers gemessen.

### Verwendete Seren:

Es wurden die Zöliakieseren mit der Serumnummer 1011, 1020, 1038 sowie 1045 verwendet.
Die Seren 1011 sowie 1038 wurden 1:10 mit einem Blutspenderserum vorverdünnt und schließlich nochmal 1:100 mit Probenpuffer.
Das Maß der Kompetition wurde bei jedem Serum im Vergleich zum Referenzwert berechnet. Der Referenzwert wurde auf 100 % gesetzt und Diagramme mit Hilfe von Microsoft Office Excel erstellt.

### Ergebnisse des Kompetitionstests IgA bzw. IgG:

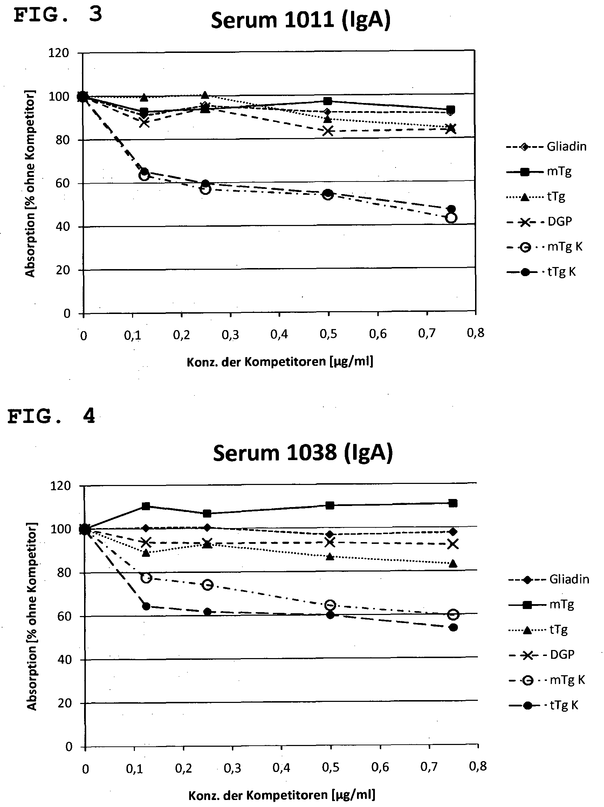

Es ist zu erkennen, dass Antikörper durch die einzelnen Komponenten abgefangen werden vgl. Seren 1011 sowie 1038 (FIG. 3 bzw. FIG. 4 für IgA und FIG. 5 bzw. FIG. 6 für IgG). In allen vier Fällen hat die Zugabe der Komponenten DGP, Gliadin sowie mTg kaum Einfluss auf die Farbintensität der Reaktion, sodass davon ausgegangen werden kann, dass die Antikörper in den Patientenseren ausschließlich gegen den erfindungsgemäß verwendeten Komplex gerichtet sind.

### Messungen zur Bildung von Komplexen über AF4/SEC MALS

Die jeweiligen Komplexe wurden über eine Größenausschlußchromatographie (SEC)-Säule oder Assymmetrische Fluss Feld Fluss Fraktionierung AF4 aufgetrennt und über Vielwinkellichtstreuung (Multi Angle Light Scattering - MALS) analysiert.

Über die Messung mittel MALS ist ein Vergleich der in einer Probe enthaltenen Massenverteilung möglich. Mit Hilfe des Massenvergleiches der MALS soll der Umsatz der Einzelkomponenten Gliadin mit der jeweiligen Transglutaminase (mTg oder tTg) zu einem Komplex nachgewiesen werden.

Zugabe von mTg führt zu deutlich höheren Massen im Vergleich zu Gliadin ohne mTg

### ELISA Analyse von Zöliakie-Patientenseren

Die verwendeten Zöliakie Seren (N=82) sowie 33 Kontrollseren (Blutspender) wurden auf die folgenden AESKU Kits sowie auf selbst beschichtete Platten getestet.

Die Seren wurden auf IgA sowie auf IgG Antikörper gegen die einzelnen Komponenten oder Komplexe getestet.

AESKU Kits:
- AESKULISA Glia
- AESKULISA DGP
- AESKULISA tTg
- AESKULISA tTg New Generation

Selbst beschichtete Platten:
- Mikrobielle Transglutaminase
- Gliadin in Wasser
- Gliadin mit mikrobieller Transglutaminase
- Aufgereinigter mTg - Gliadin Komplex

Die Ergebnisse der ELISA sind in der Tabelle 1 zusammengefasst. Die Konzentration der Proteinlösungen wurde mittels der OD bei 280nm bestimmt (Annahme: 1 OD = 1µg/ml).

**Tabelle 1: Auszüge der ROC-Analyse.**

| Marker | AUC | Sensitivität | Spezifität |
|---|---|---|---|
| AESKULISA tTg-G New Generation | 0.95 +/- 0.02 | 0.87 +/- 0.04 | 0.93 +/- 0.03 |
| AESKULISA GliaG | 0.671 +/- 0.042 | 0.65 +/- 0.06 | 0.89 +/- 0.04 |
| mTg-GP-Komplex-IgG | 0.89 +/- 0.03 | 0.91 +/- 0.03 | 1.00 +/- 0.00 |
| tTg-IgG ELISA | 0.81 +/- 0.04 | 0.6 +/- 0.06 | 0.94 +/- 0.03 |
| mTg-IgG ELISA | nicht bestimmbar | | |
| AESKULISA DGP IgG | 0.73 +/- 0.04 | 0.58 +/- 0.06 | 0.79 +/- 0.05 |
| AESKULISA tTg-A New Generation | 0.95 +/- 0.02 | 0.89 +/- 0.04 | 0.93 +/- 0.03 |
| AESKULISA Glia A | 0.77 +/- 0.04 | 0.67 +/- 0.06 | 0.81 +/- 0.04 |
| mTg-Komplex-IgA | 0.90 +/- 0.03 | 0.69 +/- 0.054 | 1.00 +/- 0.00 |
| tTg-IgA ELISA | 0.87 +/- 0.03 | 0.62 +/- 0.06 | 1.00 +/- 0.00 |
| mTg-IgA ELISA | nicht bestimmbar | | |
| AESKULISA DGP IgA | 0.68 +/- 0.04 | 0.41 +/- 0.06 | 0.96 +/- 0.02 |

Da für mTg-ELISAs keine Transformation in andere Einheiten möglich ist (nicht etablierte Tests) basieren diese Analyse auf den optischen Dichten der Assays, wobei das Sensitivitäts-/Spezifitätsverhältnis so optimiert wurde, dass ihre Summe maximal ist (sog. Youden-Index).

Aufgrund des Auswahl-Bias werden diese Werte von denen einer klinischen Studie abweichen.
Wie die Tabelle 1 belegt, verhalten sich die mTg-Komplex-ELISAs anders als die anderen Parameter und auch anders als die tTG-Gliadin-Komplex-ELISAs. Anzumerken ist, dass eine Unterscheidung über die Antikörper gegen die reine mTg nicht möglich ist. Zu beachten ist, dass die tTG2-Gliadin-Komplex-ELISAs zwar signifikant besser abschneiden, als die mTg-Gliadin-Komplex-ELISAs, diese aber wiederrum besser als die übrigen ELISAs - obwohl die Patienten kaum auf die mTg direkt ansprechen. Wie bereits ausgeführt, ist daher von einer Epitopähnlichkeit zwischen tTG2-Gliadin-Komplexen und mTg-Gliadin-Komplexen auszugehen - jedoch nicht zwingend einer Epitopgleichheit.

Stellt der erfindungsgemäß verwendete Komplex das erste immunopotente Epitop für eine Zöliakieneuerkrankung, so kann molekulare Epitopmimikrie dazu führen, dass das Patientenimmunsystem in der Folge Antikörper gegen körpereigene Gewebs-Transglutaminase im Komplex mit Gliadin und in der weiteren Folge - als Konsequenz sekundärer molekularer Epitopmimikrie - gegen Epitope, die sich auch der Gewebs-Transglutaminase, Gliadin, Gliadinpeptiden oder deamidierten Derivaten (DGPs = Deamidierte Gliadinpeptide) rekrutieren. Zöliakiepatienten scheinen nicht zu jedem Zeitpunkt ihren Antikörpertiter gegen bestimmte Epitope zu bilden, sondern es scheinen sich transiente Übergänge von einem der geschilderten Epitope zu bilden was durchaus unter Beibehaltung von "Resttitern" den Krankheitsverlauf kennzeichnet, insbesondere auch, da eine glutenfreie Diät zu einem geringeren Titer der etablierten Antikörper führen kann.

Um diesen Sachverhalt nachzuweisen, wurde an einem vorhandenen Serenpanel (75 unabhängige Zöliakiepatientenserumproben verschiedener Nationalitäten) ein multipler Wilcoxon-Mann-Whitney-Test (auch U-Test) als Rangsummentest durchgeführt und die erhaltenen P-Werte als Maß für die die Unterschiede der Rangverteilung gezeigt. Hierbei wird der P-Wert - analog zum sog. Manhattan Plot - logarithmiert und im Vorzeichen invertiert (je ausgeprägter der Rangsummenunterschied der Parameterpaare, desto höher der Balken), wobei der horizontale Balken dem Bonferronisignifikanzschwellenwert, als möglichst konservative Korrektur für multiples Testen, entspricht.
Gemäß dem oben beschriebenen Sachverhalt ist davon auszugehen, dass analoge Epitop-Paare (z. B. Anti-Gliadin-Antikörper vs. DGP-Antikörper oder Anti-mTgase-Gliadinpeptidkomplex-Antikörper vs. Anti-humane-Gewebstransglutaminase-Komplex-Antikörper) gleichförmigere Rangsummen (kleinere P-Wertbalken) aufweisen als Epitope, deren Titerhöhe mit dem Erkrankungsverlauf korrelieren werden (z. B. Anti-mTgase-Gliadinpeptidkomplex-Antikörper vs. Anti-Gliadin-Antikörper).

Durch Detektion dieser Antikörper könnten auch Zöliakie- oder Sprue Patienten identifiziert werden, die mit einem oder mehreren anderen serologischen Diagnosetests für Zöliakie oder Sprue nicht erkannt werden. Dadurch lassen sich mit der erfindungsgemäßen Verwendung auch solche Patienten identifizieren, welche zuvor als falsch-negativ eingestuft wurden. Dies betrifft in unserem Panel von 75 zöliakiepositiven Seren insgesamt 8 Seren beim Anti-tTg-Gliadinkomplex-Antikörpertest, 25 Seren beim Anti-Gliadin-Antikörpertest, 44 Seren beim Anti-DGP-Antikörpertest und 28 Seren beim Anti-tTg-Antikörpertest bzgl. der IgA-Antikörper. Die damit zu schließende diagnostische Lücke ist somit hoch signifikant (P-Wert von 7,4 * 10e-8 bei einem Cochran-Armitage-Trendtest auf den Trend des erfindungsgemäß verwendeten Komplexes). Mit Einführung des im Antrags angesprochenen Kits kann die geschlossene Lücke noch vergrößert werden, da das betrachtete Serenpanel einem Auswahlbias unterliegt, der zu ohnehin positiv befundeten Patienten neigt.

Das Auffinden des erfindungsgemäß verwendeten Komplexes ist umso überraschender, da zwischen mikrobieller Transglutaminase und Gewebstransglutaminase nahezu keine Homologie vorhanden ist.

## Patentansprüche

1. In vitro-Verwendung einer immunologisch reaktiven mikrobiellen Transglutaminase zur Diagnose und/oder Therapiekontrolle von Zöliakie oder Sprue sowie von Glutensensitivität, **dadurch gekennzeichnet, dass** ein immunologisch reaktiver Komplex, gebildet aus der mikrobiellen Transglutaminase oder Teile derselben und Gliadin oder Teilpeptiden desselben zum Nachweis von Antikörpern aus einer Probe dient und diese Antikörper nachweisbar sind, noch bevor Antikörper nachweisbar sind, die gegen einen Komplex aus Gewebstransglutaminase und Gliadin gebildet werden.

2. In vitro-Verwendung nach Anspruch 1, **dadurch gekennzeichnet, dass** die mikrobielle Transglutaminase eine Aminosäuresequenz oder Teile derselben mit der SEQ. ID. Nr. 1 aufweist.

3. In vitro-Verwendung nach einem der vorhergehenden Ansprüche, **dadurch gekennzeichnet, dass** das Gliadin eine Aminosäuresequenz oder Teile derselben mit der SEQ. ID. Nr. 2 aufweist.

4. In vitro-Verwendung nach einem der vorhergehenden Ansprüche, **dadurch gekennzeichnet, dass** die mikrobielle Transglutaminase der Gattung *Streptomyces* angehört.

5. In vitro-Verwendung nach einem der vorhergehenden Ansprüche, **dadurch gekennzeichnet, dass** zum Nachweis der an den immunologisch reaktiven Komplex gebundenen Antikörper aus einer Probe ein Immunassay-Verfahren unter Verwendung von Antikörpern zum Nachweis von an den immunologisch reaktiven Komplex gebundenen Antikörpern aus einer Probe verwendet wird.

6. In vitro-Verwendung nach Anspruch 5, **dadurch gekennzeichnet, dass** die Antikörper zum Nachweis spezifisch für den Isotyp IgA, IgM, IgE und/oder IgG sind.

7. In vitro-Verwendung nach Anspruch 5, **dadurch gekennzeichnet, dass** das Immunassay-Verfahren ein ELISA, RIA oder Fluoreszenzimmunoassay ist, und/oder dass der Nachweis an einer festen oder in einer flüssigen Phase durchgeführt wird.

8. Kit zur Bestimmung von Diagnose und/oder Therapiekontrolle von Zöliakie oder Sprue sowie von Glutensensitivität, **dadurch gekennzeichnet, dass** der Kit einen immunologisch reaktiven Komplex aus einer mikrobiellen Transglutaminase oder einem Teil derselben und Gliadin oder einem Teilpeptid desselbigen aufweist, wobei der immunologisch reaktive Komplex Nachweis von Antikörpern aus einer Probe dient und diese Antikörper nachweisbar sind, noch bevor Antikörper nachweisbar sind, die gegen einen Komplex aus Gewebstransglutaminase und Gliadin gebildet werden und der Kit zusätzliche Mittel zum spezifischen Nachweis von an den immunologisch reaktiven Komplex gebundenen Antikörpern aus einer Probe umfasst.

9. Kit nach Anspruch 8, **dadurch gekennzeichnet, dass** die mikrobielle Transglutaminase eine Aminosäuresequenz oder Teile derselben mit der SEQ. ID. Nr. 1 aufweist.

10. Kit nach Anspruch 8 oder 9, **dadurch gekennzeichnet, dass** das Gliadin eine Aminosäuresequenz oder Teile derselben mit der SEQ. ID. Nr. 2 aufweist.

11. Kit nach Anspruch 8 bis 10, **dadurch gekennzeichnet, dass** die mikrobielle Transglutaminase der Gattung *Streptomyces* angehört.

12. Kit nach einem der Ansprüche 8 bis 11, **dadurch gekennzeichnet, dass** die zusätzlichen Mittel zum spezifischen Nachweis Antikörper für den Isotyp IgA, IgM, IgE und/oder IgG sind.

13. Kit nach einem der Ansprüche 8 bis 12, **dadurch gekennzeichnet, dass** der Kit zusätzliche Mittel zur Einteilung von Zöliakie und/oder Sprue sowie von Glutensensitivität in verschiedene Untergruppen der Krankheiten wie tTg-Antikörper, tTg/Gliadinpeptid-Komplex-Antikörper, DGP-Antikörper, Gliadin-Antikörper usw. umfasst.

14. Kit nach einem der Ansprüche 8 bis 13, **dadurch gekennzeichnet, dass** der Kit zusätzlich isolierte monoklonale und/oder polyklonale und/oder rekombinante Antikörper, welche spezifisch an einen immunologisch reaktiven Komplex aus einer mikrobiellen Transglutaminase oder einem Teil derselben und Gliadin oder einem Teilpeptid desselbigen binden, enthält.

15. Kit nach einem der Ansprüche 8 bis 14, **dadurch gekennzeichnet, dass** der immunologisch reaktive Komplex gebildet aus einer mikrobiellen Transglutaminase oder einem Teil derselben und Gliadin und/oder einem Teilpeptid desselbigen vorliegt.

## Claims

1. In vitro use of an immunologically reactive microbial transglutaminase for diagnosis and/or monitoring of the treatment of celiac disease or sprue as well as gluten sensitivity, **characterized in that** an immunologically reactive complex formed from the microbial transglutaminase or parts thereof and gliadin or partial peptides thereof is used for detection of antibodies from a sample and these antibodies are detectable even before antibodies produced against a complex of tissue transglutaminase and gliadin are detectable.

2. The in vitro use according to claim 1, **characterized in that** the microbial transglutaminase comprises an amino acid sequence or parts thereof with SEQ. ID. No. 1.

3. The in vitro use according to any one of the preceding claims, **characterized in that** the gliadin comprises an amino acid sequence or parts thereof with SEQ. ID. No. 2.

4. The in vitro use according to any one of the preceding claims, **characterized in that** the microbial transglutaminase belongs to the genus *Streptomyces.*

5. The in vitro use according to any one of the preceding claims, **characterized in that** for detecting the antibodies bound to the immunologically reactive complex from a sample, an immunoassay method using antibodies for detecting antibodies bound to the immunologically reactive complex from a sample is employed.

6. The in vitro use according to claim 5, **characterized in that** the antibodies for detection are specific for the isotype IgA, IgM, IgE and/or IgG.

7. The in vitro use according to claim 5, **characterized in that** the immunoassay method is an ELISA, RIA or fluorescence immunoassay, and/or that the detection is conducted on a solid phase or in a liquid phase.

8. A kit for determination of the diagnosis and/or monitoring of treatment of celiac disease or sprue as well as of gluten sensitivity, **characterized in that** the kit comprises an immunologically reactive complex from a microbial transglutaminase or a part thereof and gliadin or a partial peptide thereof, wherein the immunologically reactive complex is used for the detection of antibodies from a sample and these antibodies are detectable even before antibodies against a complex of tissue transglutaminase and gliadin are produced and the kit comprises additional means for specific detection of antibodies bound to the immunologically reactive complex from a sample.

9. The kit according to claim 8, **characterized in that** the microbial transglutaminase comprises an amino acid sequence or parts thereof with SEQ. ID. No. 1.

10. The kit according to claim 8 or 9, **characterized in that** the gliadin comprises an amino acid sequence or parts thereof with SEQ. ID. No. 2.

11. The kit according to claims 8 to 10, **characterized in that** the microbial transglutaminase belongs to the genus *Streptomyces.*

12. The kit according to one of claims 8 to 11, **characterized in that** the additional means for specific detection are antibodies for the isotype IgA, IgM, IgE and/or IgG.

13. The kit according to one of the claims 8 to 12, **characterized in that** the kit comprises additional means for classifying celiac disease and/or sprue as well as gluten sensitivity into various subgroups of these diseases such as tTg antibodies, tTg/gliadin peptide complex antibodies, DGP antibodies, gliadin antibodies etc.

14. The kit according to one of the claims 8 to 13, **characterized in that** the kit additionally contains isolated monoclonal and/or polyclonal and/or recombinant antibodies that specifically bind to an immunologically reactive complex from a microbial transglutaminase or a part thereof and gliadin or a partial peptide of the same.

15. The kit according to one of claims 8 to 14, **characterized in that** the immunologically reactive complex formed from a microbial transglutaminase or a part thereof and gliadin and/or a partial peptide thereof is present.

## Revendications

1. Utilisation *in vitro* d'une transglutaminase microbienne immunologiquement réactive pour le diagnostic et/ou le contrôle thérapeutique de la maladie coeliaque ou sprue ainsi que de la sensibilité au gluten, **caractérisée en ce qu'**un complexe immunologiquement réactif, formé de la transglutaminase microbienne ou de parties de celle-ci et de gliadine ou de peptides partiels de celle-ci sert pour la détection d'anticorps d'un échantillon et ces anticorps sont détectables, avant même que des anticorps qui sont formés contre un complexe de transglutaminase tissulaire et de gliadine ne soient détectables.

2. Utilisation *in vitro* selon la revendication 1, **caractérisée en ce que** la transglutaminase microbienne présente une séquence d'acides aminés ou des parties de celle-ci avec la SEQ ID N° : 1.

3. Utilisation *in vitro* selon l'une quelconque des revendications précédentes, **caractérisée en ce que** la gliadine présente une séquence d'acides aminés ou des parties de celle-ci avec la SEQ ID N° : 2.

4. Utilisation *in vitro* selon l'une quelconque des revendications précédentes, **caractérisée en ce que** la transglutaminase microbienne appartient au genre *Streptomyces.*

5. Utilisation *in vitro* selon l'une quelconque des revendications précédentes, **caractérisée en ce que** pour la détection des anticorps d'un échantillon liés au complexe immunologiquement réactif, un procédé d'essai immunologique utilisant des anticorps pour la détection d'anticorps d'un échantillon liés au complexe immunologiquement réactif est utilisé.

6. Utilisation *in vitro* selon la revendication 5, **caractérisée en ce que** les anticorps pour la détection sont spécifiques de l'isotype IgA, IgM, IgE et/ou IgG.

7. Utilisation *in vitro* selon la revendication 5, **caractérisée en ce que** le procédé d'essai immunologique est un essai ELISA, RIA ou un essai immunologique à fluorescence, et/ou **en ce que** la détection est réalisée en phase solide ou en phase liquide.

8. Kit pour la détermination de diagnostics et/ou pour le contrôle thérapeutique de la maladie coeliaque ou sprue ainsi que de la sensibilité au gluten, **caractérisé en ce que** le kit présente un complexe immunologiquement réactif d'une transglutaminase microbienne ou d'une partie de celle-ci et de gliadine ou d'un peptide partiel de celle-ci, dans lequel le complexe immunologiquement réactif sert pour la détection d'anticorps d'un échantillon et ces anticorps sont détectables, avant même que des anticorps qui sont formés contre un complexe de transglutaminase tissulaire et de gliadine soient détectables et le kit comprend des moyens supplémentaires pour la détection spécifique d'anticorps d'un échantillon liés au complexe immunologiquement réactif.

9. Kit selon la revendication 8, **caractérisé en ce que** la transglutaminase microbienne présente une séquence d'acides aminés ou des parties de celle-ci avec la SEQ ID N° : 1.

10. Kit selon la revendication 8 ou 9, **caractérisé en ce que** la gliadine présente une séquence d'acides aminés ou des parties de celle-ci avec la SEQ ID N° : 2.

11. Kit selon la revendication 8 à 10, **caractérisé en ce que** la transglutaminase microbienne appartient au genre *Streptomyces.*

12. Kit selon l'une quelconque des revendications 8 à 11, **caractérisé en ce que** les moyens supplémentaires pour la détection spécifique sont des anticorps pour l'isotype IgA, IgM, IgE et/ou IgG.

13. Kit selon l'une quelconque des revendications 8 à 12, **caractérisé en ce que** le kit présente des moyens supplémentaires pour la classification de la maladie coeliaque et/ou sprue ainsi que de la sensibilité au gluten en différents sous-groupes de maladies tels que des anticorps tTg, des anticorps du complexe tTg/peptide de gliadine, des anticorps DGP, des anticorps de la gliadine etc.

14. Kit selon l'une quelconque des revendications 8 à 13, **caractérisé en ce que** le kit contient en outre des anticorps monoclonaux et/ou polyclonaux et/ou recombinants isolés qui se lient spécifiquement à un complexe immunologiquement réactif d'une transglutaminase microbienne ou d'une partie de celle-ci et de gliadine ou à un peptide partiel de celle-ci.

15. Kit selon l'une quelconque des revendications 8 à 14, **caractérisé en ce que** le complexe immunologiquement réactif est formé d'une transglutaminase microbienne ou d'une partie de celle-ci et de gliadine et/ou d'un peptide partiel de celle-ci.


### Sequenzprotokoll

<110> AESKU.Diagnostics GmbH & Co.KG Verfahren zur Präsymptomatischen Diagnostik
<120> von Zöliakie und Glutensensitivität
<160> 2
<210> 1
   <211> 410
   <212> Protein
   <213> Streptomyces mobaraensis
<400> 1
   Met Ser Gln Arg Gly Arg Thr Leu Val Phe Ala
   Ala Leu Gly Ala Val Met Cys Thr Thr Ala Leu
   Met Pro Ser Ala Gly Ala Ala Thr Gly Ser Gly
   Ser Gly Ser Gly Thr Gly Glu Glu Lys Arg Ser
   Tyr Ala Glu Thr His Arg Leu Thr Ala Asp Asp
   Val Asp Asp Ile Asn Ala Leu Asn Glu Ser Ala
   Pro Ala Ala Ser Ser Ala Gly Pro Ser Phe Arg
   Ala Pro Asp Ser Asp Glu Arg Val Thr Pro Pro
   Ala Glu Pro Leu Asp Arg Met Pro Asp Pro Tyr
   Arg Pro Ser Tyr Gly Arg Ala Glu Thr Ile Val
   Asn Asn Tyr Ile Arg Lys Trp Gln Gln Val Tyr
   Ser His Arg Asp Gly Arg Lys Gln Gln Met Thr
   Glu Glu Gln Arg Glu Trp Leu Ser Tyr Gly Cys
   Val Gly Val Thr Trp Val Asn Ser Gly Gln Tyr
   Pro Thr Asn Arg Leu Ala Phe Ala Phe Phe Asp
   Glu Asp Lys Tyr Lys Asn Glu Leu Lys Asn Gly
   Arg Pro Arg Ser Gly Glu Thr Arg Ala Glu Phe
   Glu Gly Arg Val Ala Lys Asp Ser Phe Asp Glu
   Ala Lys Gly Phe Gln Arg Ala Arg Asp Val Ala
   Ser Val Met Asn Lys Ala Leu Glu Asn Ala His
   Asp Glu Gly Ala Tyr Leu Asp Asn Leu Lys Lys
   Glu Leu Ala Asn Gly Asn Asp Ala Leu Arg Asn
   Glu Asp Ala Arg Ser Pro Phe Tyr Ser Ala Leu
   Arg Asn Thr Pro Ser Phe Lys Asp Arg Asn Gly
   Gly Asn His Asp Pro Ser Lys Met Lys Ala Val
   Ile Tyr Ser Lys His Phe Trp Ser Gly Gln Asp
   Arg Ser Gly Ser Ser Asp Lys Arg Lys Tyr Gly
   Asp Pro Glu Ala Phe Arg Pro Asp Arg Gly Thr
   Gly Leu Val Asp Met Ser Arg Asp Arg Asn Ile
   Pro Arg Ser Pro Thr Ser Pro Gly Glu Ser Phe
   Val Asn Phe Asp Tyr Gly Trp Phe Gly Ala Gln
   Thr Glu Ala Asp Ala Asp Lys Thr Val Trp Thr
   His Gly Asn His Tyr His Ala Pro Asn Gly Ser
   Leu Gly Ala Met His Val Tyr Glu Ser Lys Phe
   Arg Asn Trp Ser Asp Gly Tyr Ser Asp Phe Asp
   Arg Gly Ala Tyr Val Val Thr Phe Val Pro Lys
   Ser Trp Asn Thr Ala Pro Asp Lys Val Lys Gln
   Gly Trp Pro
<110> AESKU.Diagnostics GmbH & Co.KG
<120> Verfahren zur Präsymptomatischen Diagnostik von Zöliakie und Glutensensitivität
<160> 2
<210> 2
   <211> 286
   <212> Protein
   <213> Triticum aestivum
<400> 2
   Met Lys Thr Phe Leu Ile Leu Val Leu Leu Ala
   Ile Val Ala Thr Thr Ala Thr Thr Ala Val Arg
   Phe Pro Val Pro Gln Leu Gln Pro Gln Asn Pro
   Ser Gln Gln Gln Pro Gln Glu Gln Val Pro Leu
   Val Gln Gln Gln Gln Phe Leu Gly Gln Gln Gln
   Pro Phe Pro Pro Gln Gln Pro Tyr Pro Gln Pro
   Gln Pro Phe Pro Ser Gln Leu Pro Tyr Leu Gln
   Leu Gln Pro Phe Pro Gln Pro Gln Leu Pro Tyr
   Ser Gln Pro Gln Pro Phe Arg Pro Gln Gln Pro
   Tyr Pro Gln Pro Gln Pro Gln Tyr Ser Gln Pro
   Gln Gln Pro Ile Ser Gln Gln Gln Gln Gln Gln
   Gln Gln Gln Gln Gln Gln Gln Gln Gln Gln Gln
   Gln Ile Leu Gln Gln Ile Leu Gln Gln Gln Leu
   Ile Pro Cys Met Asp Val Val Leu Gln Gln His
   Asn Ile Ala His Gly Arg Ser Gln Val Leu Gln
   Gln Ser Thr Tyr Gln Leu Leu Gln Glu Leu Cys
   Cys Gln His Leu Trp Gln Ile Pro Glu Gln Ser
   Gln Cys Gln Ala Ile His Asn Val Val His Ala
   Ile Ile Leu His Gln Gln Gln Lys Gln Gln Gln
   Gln Pro Ser Ser Gln Val Ser Phe Gln Gln Pro
   Leu Gln Gln Tyr Pro Leu Gly Gln Gly Ser Phe
   Arg Pro Ser Gln Gln Asn Pro Gln Ala Gln Gly
   Ser Val Gln Pro Gln Gln Leu Pro Gln Phe Glu
   Glu Ile Arg Asn Leu Ala Leu Gln Thr Leu Pro
   Ala Met Cys Asn Val Tyr Ile Pro Pro Tyr Cys
   Thr Ile Ala Pro Phe Gly Ile Phe Gly Thr Asn